# EUROPEAN PATENT SPECIFICATION

(11) **EP 2 814 415 B1**
(45) Date of publication and mention of the grant of the patent: **07.06.2023**
(21) Application number: 13709981.8
(22) Date of filing: 18.02.2013
(51) Int. Cl.: A61B 18/14, A61B 18/00

(54) **ELECTROSURGICAL IMPLEMENT**
ELEKTROCHIRURGISCHE VORRICHTUNG
INSTRUMENT ÉLECTROCHIRURGICAL

(30) Priority: 17.02.2012 GB 201202759; 17.02.2012 US 201261600083 P
(43) Date of publication of application: 24.12.2014
(73) Proprietor: Brooke, Gerard Michael, Stroud, Gloucestershire GL5 3QF (GB)
(72) Inventor: Brooke, Gerard Michael, Stroud, Gloucestershire GL5 3QF (GB)
(74) Representative: Hocking, Adrian Niall
(86) International application number: PCT/GB2013/050385
(87) International publication number: WO 2013/121226

(56) References cited:
- EP-A1- 1 800 612
- US-A1- 2004 172 018
- US-A1- 2006 079 882
- US-A1- 2007 049 922
- US-A1- 2007 112 343
- US-A1- 2007 112 343
- US-A1- 2008 125 775
- US-A1- 2010 217 073
- US-A1- 2011 319 709
- US-B1- 6 428 538
- US-B1- 8 079 982
- US-B2- 7 220 267

## Description

This invention relates to electrosurgical implements and in particular, but not exclusively, to single use disposable electrosurgical implements as defined in independent claim 1. Preferred embodiments are disclosed in the dependent claims.

In electrosurgical procedures, a radio frequency current (RF), otherwise known as an electrosurgery current, is passed by an electrode into tissue to effect cutting or cauterisation, depending on the magnitude and wave form of the applied current. Conventional electrosurgical implements are of pen-like form, comprising an elongate tubular body of insulating material, from the rear (proximal) end of which passes a conductive cable to an electrosurgery generator. Relevant prior art can be found in US2007/112343.

Prior to use, an electrode which may be re-usable or single use, is selected and plugged into an orifice at the front (distal) end of the body to make electrical contact with an electrical circuit contained in the body of the instrument, and operated by one or more buttons on the instrument body.

The patient contact region of the electrode may take various forms according to the nature of the operation to be conducted. In such devices it is intended that the electrode be replaced after each use, not only to prevent the possibility of contamination or cross-infection, but also because the treatment surfaces can become eroded by electrolytic action thereby blunting the electrode. In addition, once an electrode has been fitted into the front end of the implement there is no protection for the electrode against physical damage prior to its use. The body of the device may be re-useable or single use and, in the
case of the re-useable product, is designed to be used many times and will require cleaning and sterilisation after each use.

In the operating theatre environment it is awkward and potentially hazardous to fit an electrode into the body, and where the electrode has a sharp tip or cutting edge, there is the risk of injury.

Accordingly, in one aspect, this invention provides an electrosurgical implement comprising:
an elongate body having an opening at its front end and comprising a rotatable rear barrel portion and a pressing which is fixed both rotationally and longitudinally with respect to the rear barrel portion;
an electrode assembly comprising a treatment electrode through which a treatment current may be passed in use, the treatment electrode having a patient contact region at its forward end;
an electrode holder which carries the electrode assembly being carried in said body, the electrode holder being movable between a retracted position in which the patient contact region of the electrode is shielded within said elongate body and at least one extended position in which said patient contact region projects through said opening;
a bias arrangement for rearwardly biasing said electrode assembly towards its retracted position,
a latch arrangement for latching the electrode holder comprising a rear latch tooth engagable with at least one aperture of the pressing, the latch arrangement holding the electrode holder in the at least one extended position when a user presses rearwardly on an exposed portion of the electrode holder so as to hold the electrode assembly in said at least one extended position; and
the rear barrel being rotatable relative to the remainder of the elongate body to urge the rear latch tooth into alignment with a longitudinally cutaway slot in the pressing, to unlatch the electrode holder for rearward retraction movement under the influence of the bias arrangement.

By this arrangement, the patient contact region of the electrode may be shielded within the elongate body prior to use, and extended only when required. Preferably the body is of generally tubular form; the term tubular is used to mean a body of hollow section which may be uniform or which may vary along its length. The cross section may be circular, elliptical, polygonal or any other suitable shape.

Preferably said implement is configured to provide two or more extended positions for said electrode assembly.

This gives the surgeon a choice over the amount by which the patient contact region projects from the forward end of the electrosurgical implement. This means that a single implement can be used to provide different lengths of projection which, in a conventional device, would require one electrode to be changed for another.

The electrode assembly may be moved within the body by various externally operated means connected directly or indirectly to the electrode assembly, for example by a slide member accessible through a slot in the body, or by a slideable sleeve carried on the outside of the body. But it is preferred for the electrode assembly to comprise a rearward member which is non-conducting or which is insulated from the current passing to the electrode assembly, which projects rearwardly from the elongate body and which may be pushed forwardly to move the electrode assembly to its extended position or positions. This provides a simple intuitive operation and allows the sides of the tubular housing to be left unobstructed save for operating switches etc.

Although the patient contact region of the electrode could be protected after use by capping the exposed patient contact region, it is preferred for there to be a release arrangement which is operable to unlatch or otherwise release the electrode assembly for rearward retraction movement under the influence of said bias arrangement.

The latch or holding arrangement may conveniently comprise cooperating elements associated with the electrode assembly and said body respectively. These elements may be integrally formed on the electrode assembly and the body or one or both may be elements attached thereto or otherwise associated therewith, such as inserts.

Preferably, the latch arrangement comprises a tooth associated with or provided on the electrode assembly, and a latch recess associated with said body for being engaged by said latch tooth when the electrode assembly is in an extended position, although an inverse arrangement in which the tooth is associated with the body is also possible.

The latch arrangement may include a pre-use recess adapted to receive said tooth when said electrode assembly is in its retracted position. The tooth and/or the recess may be resiliently movable with the tooth shaped to be moveable in a forward direction to move out of said recess by deflection of the tooth and/or recess. The tooth and/or the recess may be resiliently moveable so that the tooth may snap into a recess when the electrode assembly is moved forwards to an extended position. If there is more than one extended position the tooth may move out of the first extended recess by deflection as aforesaid, then snap into the second extended recess.

The disclosure also extends to an electrosurgical implement comprising a body, an electrode assembly mounted for longitudinal movement between retracted and extended positions relative to the body, a releasable holding arrangement for holding the electrode assembly in an extended position, and releasable to allow the electrode to be retracted into the body. The implement may include a bias for biasing the electrode assembly towards a retracted position. The implement may include a lock which operates to retain electrode assembly in its retracted position after use.

Preferably, the relative angular movement of the electrode assembly and at least part of said body provides movement between an operative position in which said cooperating elements are in longitudinal alignment in which retraction of the electrode is prevented and a release position in which the cooperating elements are out of alignment, thereby allowing the electrode assembly to be retracted under the influence of the bias arrangement. The biasing arrangement may be a spring.

Preferably at least one of the electrode assembly and the body is configured so that, on effecting said angular movement between said electrode assembly and said body into said release position, said electrode assembly, having retracted into the housing after use, is biased by the biasing means so as to prevent it from moving back forwardly to a position in which the patient contact region would be re-exposed.

In one particularly preferred embodiment, a further latching arrangement is provided between the electrode assembly and the body which operates to latch the electrode assembly in its retracted position permanently or semi- permanently.

Preferably said body includes a forward section and a rearward section, with the rearward section being angularly movable relative to the forward section between said operative position and said release position.

By way of example only, two specific embodiments of electrosurgical pencil will now be described, with reference being made to the accompanying drawings, in which:
Figure 1 is a general perspective view of a first embodiment of this invention;
Figures 2(a) and (b) are top and side section views respectively of the embodiment of Figure 1;
Figures 3(a) and (b) to Figures 7(a) and (b) are respective top and side section views through the embodiment of Figures 1 and 2 in a primed position, in a first operating position, a second operating position, a release position and retracted position respectively;
Figure 8 is an exploded view of the embodiment of Figures 1 to 7;
Figure 9 is an enlarged perspective view of the pressing used in the embodiment of Figures 1 to 8, showing the latch in its primed, first, second and deactive positions;
Figure 10 is a view of a single button variant of the embodiment of the invention in its pre-use position (with the rear barrel removed);
Figure 11 is a view of the single button variant in a first operating position (with the rear barrel removed), and
Figure 12 is a view of the single button variant in a second operating position (with the rear barrel removed).

The embodiments shown in the drawings are closely related and are identical in construction save for the provision of two operating buttons on the first described embodiment ('cut' and 'cauterised'), and the provision of just a single 'cut' button on the variant. The electrosurgical pencils are single use, disposable devices incorporating an extendable and retractable electrode assembly therein. The pencils in use are connected to a power supply using a cable extending from the rear end of the device.

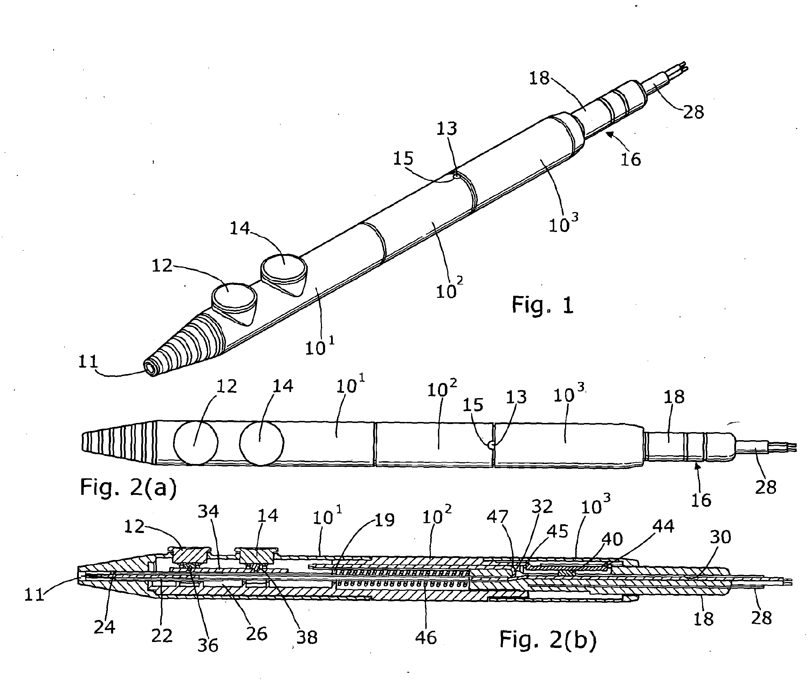

Referring initially to Figures 1 to 9, the illustrated embodiment comprises an elongate tubular body 10 made up of front, central and rear barrel portions 10¹, 10², and 10³ respectively. The forward end of the front barrel is conical and terminates in a front opening 11. The front and central barrel portions are fixed to each other and do not move during operation but the rear barrel 10³ is rotatably mounted so that it can be twisted to deactivate the device as to be described below. The rear barrel portion 10 carries a forward projection 13 that cooperates with a recess 15 in the mid-barrel portion 10² to provide a detent action.

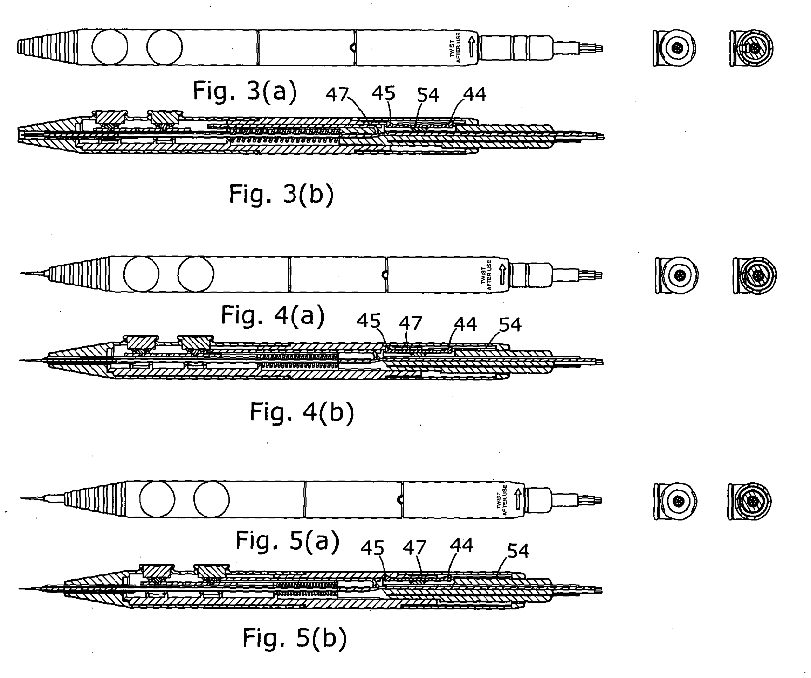

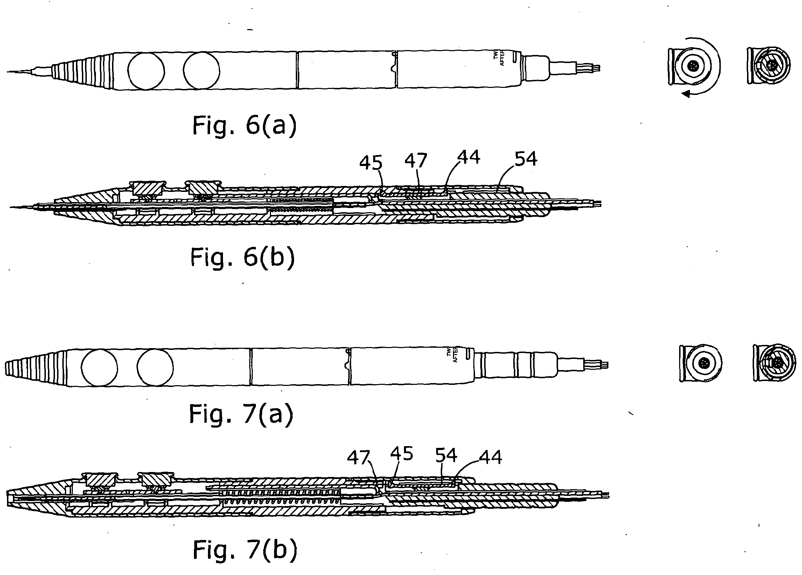

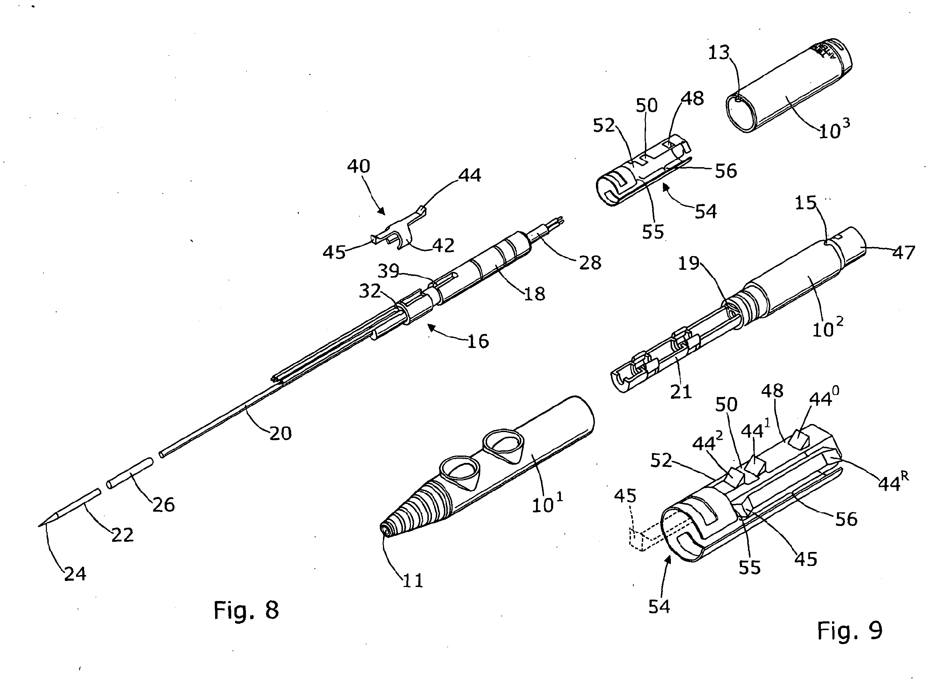

Two operating buttons 12, 14 are provided on the front barrel portion. Extending from the rear of the rear barrel portion 103 is the rear end of an electrode assembly 16 which can be seen in more detail in Figure 8. The electrode assembly 16 is mounted for longitudinal movement within the body between the pre-use position shown in Figures 2 and 3 to one or more extended positions in which a forward tip of the electrode assembly protrudes from the front end of the barrel as seen in Figures 4 to 6, and thereafter to a retracted position shown in Figure 7.

Referring more particularly to Figure 8, the electrode assembly 16 comprises an electrode holder 18 formed of non-conducting or insulating material such as plastic and is of generally cylindrical form. From the front end of the electrode holder 18 extends a conducting electrode tube 20 to which is electrically connected a needle 22 with a sharp tip 24. The rearward portion of the needle 22 and the forward portion of the tube 20 are covered by a non- conducting needle cover 26. A cable 28 passes down an axial passage 30 in the electrode holder 18 and emerges at port 32 in the forward end of the electrode holder to pass to a printed circuit board sub-assembly 34 (not shown in Figure 8). The printed circuit board sub-assembly 34 has two operating switches 36, 38 which underlie and are activated by the two buttons 12, 14. A supply lead from the printed circuit board sub-assembly passes back to the electrode holder 18 to be routed to contact the rear end of the electrode tube 20. In this manner power is supplied from a remote source to pass through the cable to the printed circuit board sub-assembly 34 and thence to the electrode tube 20 when the appropriate button is pressed.

About one third of the way back on the electrode holder is clipped in a suitable recessed area 39 a double ended latch element 40 having a split tubular clip 42 by which it may be clipped into a complementary groove on the electrode holder with a strip extending rearwardly to carry an outwardly directed rear latch tooth 44 and a similar strip extends forwardly to carry an outwardly directed forward latch tooth 45. The electrode assembly is biased rearwardly by a compression spring 46 (not shown in Figure 8, but see e.g. Figure 2) acting between the forward end of the electrode holder 18 and an internal wall 19 formed in the central barrel portion 102 The printed circuit board sub-assembly 34 is mounted in use on a forwardly extending tubular extension 21 of the central barrel portion 102 (see Figure 8). The rear sprung latch tooth 44 on the electrode holder cooperates with apertures 48, 50, 52 in a pressing 54 which is held inside the rear barrel portion 10³ and fixed both rotationally and longitudinally with respect to the rear barrel portion. The pressing 54 has a pre-use aperture 48 in which the rear latch tooth 44 sits when the device is in its pre- use position. Aligned longitudinally and forwardly of the pre-use aperture 48 are first and second apertures 50, 52 which are adapted to receive the rear latch tooth 44 to latch the electrode assembly 16 in first and second active positions when the assembly is pushed forwardly.

The forward latch tooth 45 is initially disposed under the rim of a rear collar 47 of the mid-barrel portion 102 and is radially inwardly flexed but free to slide forwardly into the mid-barrel as the electrode assembly is pushed forwardly from its primed position to each of its active positions. However, when the electrode assembly is retracted after use it moves rearwardly of the primed position sufficiently for the tooth 45 to emerge and spring out radially to latch against a latch surface 55 on the pressing and to hold the electrode assembly against forward movement from its retracted position.

Before and during use, the pressing 54 and the rear barrel portion 103 are in the orientation shown in Figure 8 with the pre-use, first and second operating apertures (48, 50, 52) in longitudinal alignment with the latch tooth 44. The rear barrel portion 103 may however be rotated clockwise (when viewed from the rear of the device) in order to rotate the rear barrel portion 103 and the pressing 54 (whilst the electrode assembly, including the latch element 40, is fixed against rotation) so that the latch tooth 44 is aligned with a longitudinally cutaway slot 56 in the pressing.

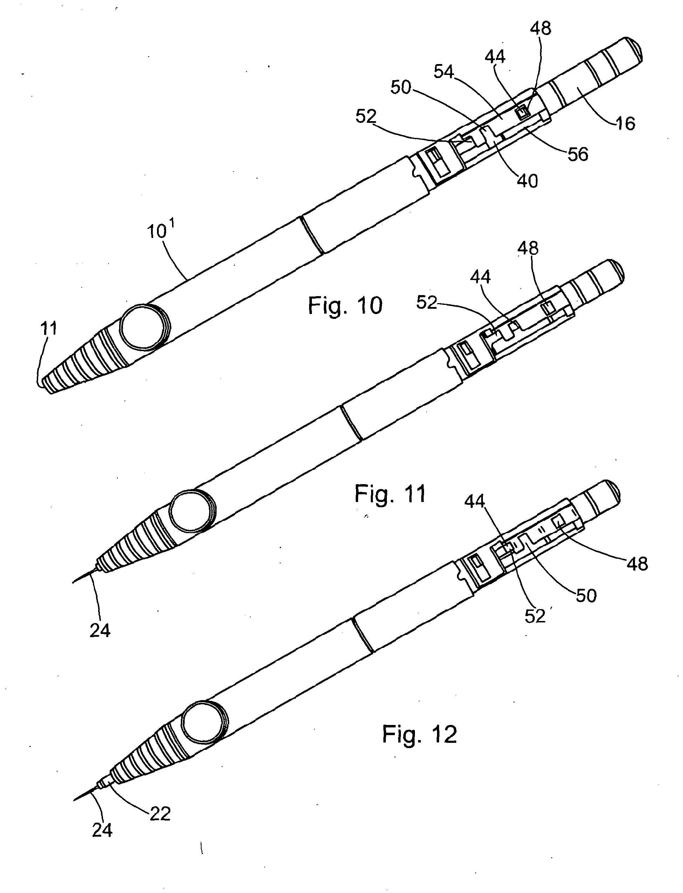

In use, the electrosurgical pencil is supplied in its pre-use position shown in Figures 2, 3 and 10, with the rear latch tooth 44 in the pre-use aperture 48, the front latch tooth 45 slideably tucked inside the rear collar 47, and the electrode needle 22 shrouded. The latch tooth 44 is shown as 44° in Figure 9. To prepare the pencil for use, the user presses rearwardly on the exposed portion of the electrode holder 18 to move the entire electrode assembly forwards to the position shown in Figures 4 and 11, with the rear latch tooth 44 in the first active aperture 50 (latch tooth shown as 441 in Figure 9). In this condition the electrode needle 22 projects from the front end of the device and the device is ready for use in the usual manner. If a greater extension is required, then the user can push once again on the rear end of the electrode holder 18 to move the electrode assembly further forward to the position shown in Figures 5 and Figure 12, with the rear latch tooth 44 in the forward latch aperture 52 (shown as 442 in Figure 9). The rear end of the electrode assembly 16 may carry grooves or other similar indicia to indicate to the user the amount of projection of the electrode needle 22. It will be appreciated that further latch apertures could be provided or other ratchet or similar means that provide a number of discrete detent positions.

Once the user has finished with the device it may be rendered safe by simply twisting the rear barrel portion 103 relative to the remainder of the body.

This rotates the pressing 54 relative to the latch tooth 44 so that the latch tooth is now aligned with the longitudinal cut away slot 56 and so that, under the influence of the compression spring 46, the entire electrode assembly 16 can shift rearwardly back to a safe position, with the electrode needle 22 fully retracted. In this position the electrode assembly is further back than when in the primed position, and the front latch tooth 45 emerges from its containment by the collar and latches against latch surface 55, as shown in Figures 7, 8 and 9. In this condition the rear latch tooth is shown as 44R in Figure 9; the tooth is to the rear of the initial tooth position, but this is not essential. In this manner, the electrode assembly is locked against re-extension, thereby shrouding the used electrode needle within the housing to make the device safe.

Figures 10 to 12 show a second embodiment in the respective pre-use and first and second positions respectively. The mechanism is similar in construction and operation to the earlier embodiment and the embodiment and the components will not be described in detail again. In the pre-use position the latch tooth cannot be rotated into the cut away slot 56, but once shifted forwards into one of the first and second positions, the electrode assembly can be rotated into the slot 56, so allowing the electrode assembly to retract under spring bias and to latch out in a safe position.

## Claims

1. An electrosurgical implement comprising:
an elongate body (10) having an opening at a forward end and comprising a rotatable rear barrel portion (10³) and a pressing (54) which is fixed both rotationally and longitudinally with respect to the rear barrel portion (10³);
an electrode assembly (16) comprising a treatment electrode through which a treatment current may be passed in use, the treatment electrode having a patient contact region at a forward end;
an electrode holder (18) which carries the electrode assembly (16) in said body (10), the electrode holder (18) being movable between a retracted position in which the patient contact region of the electrode is shielded within said elongate body (10) and at least one extended position in which said patient contact region projects through said opening;
a bias arrangement for rearwardly biasing said electrode assembly (16) towards its retracted position, and
wherein the electrosurgical implement further comprises a latch arrangement for latching the electrode holder (18) comprising a rear latch tooth (44) engagable with at least one aperture (48, 50, 52) of the pressing (54), the latch arrangement holding the electrode holder (18) in the at least one extended position when a user presses rearwardly on an exposed portion of the electrode holder (18); and
the rear barrel (10³) being rotatable relative to the remainder of the elongate body to urge the rear latch tooth (44) into alignment with a longitudinally cutaway slot (56) in the pressing (54), to unlatch the electrode holder (18) for rearward retraction movement under the influence of the bias arrangement.

2. An electrosurgical implement according to claim 1, the electrosurgical implement being configured to provide two or more extended positions for said electrode assembly (16).

3. An electrosurgical implement according to claim 1 or claim 2, wherein said electrode assembly (16) comprises a rearward non-conducting member which projects rearwardly from the elongate body (10) and which may be pushed forwardly to move the electrode assembly (16) to its extended position or positions.

4. An electrosurgical implement according to any one of the preceding claims, wherein the latch arrangement comprises cooperating elements associated with the electrode assembly (16) and said elongate body (10) respectively.

5. An electrosurgical implement according to claim 4, wherein said cooperating elements are formed on elements attached to the electrode assembly (16) and the elongate body (10).

6. An electrosurgical implement according to any one of the preceding claims, wherein the bias arrangement comprises a compression spring (46).

## Patentansprüche

1. Elektrochirurgisches Utensil, umfassend:
einen länglichen Körper (10), der eine Öffnung an einem vorwärtigen Ende aufweist und einen drehbaren hinteren Trommelteil (10³) und einen Pressteil (54), der sowohl rotativ als auch longitudinal in Bezug auf den hinteren Trommelteil (10³) fixiert ist, umfasst,
eine Elektrodenanordnung (16), die eine Behandlungselektrode umfasst, durch die im Gebrauch ein Behandlungsstrom geführt wird, wobei die Behandlungselektrode eine Patientenkontaktregion an einem vorwärtigen Ende aufweist;
einen Elektrodenhalter (18), der die Elektrodenanordnung (16) in dem Körper (10) trägt, wobei der Elektrodenhalter (18) zwischen einer eingefahrenen Position, in der die Patientenkontaktregion der Elektrode innerhalb des länglichen Körpers (10) abgeschirmt ist, und mindestens einer ausgefahrenen Position, in der die Patientenkontaktregion durch die Öffnung vorragt, bewegbar ist,
eine Vorspannungsanordnungen zum rückwärtigen Vorspannen der Elektrodenanordnung (16) zu ihrer eingefahrenen Position hin und
wobei das elektrochirurgische Utensil weiterhin eine Einrastanordnung zum Einrasten des Elektrodenhalters (18) umfasst, die einen hinteren Einrastzahn (44) umfasst, der mit mindestens einer Öffnung (48, 50, 52) des Pressteils (4) in Eingriff gebracht werden kann, wobei die Einrastanordnung den Elektrodenhalter (18) in der mindestens einen ausgefahrenen Position hält, wenn ein Benutzer rückwärtig auf einen freigelegten Teil des Elektrodenhalters (18) drückt; und
wobei die hintere Trommel (10³) in Bezug auf den Rest des länglichen Körpers rotierbar ist, um den hinteren Einrastzahn (44) in eine Linie mit einem longitudinalen ausgeschnittenen Schlitz (56) in dem Pressteil (54) zu treiben, um den Elektrodenhalter (18) für eine rückwärtige Einfahrbewegung unter Einwirkung der Vorspannungsanordnung auszurasten.

2. Elektrochirurgisches Utensil nach Anspruch 1, wobei das elektrochirurgische Utensil konfiguriert ist, um zwei oder mehr ausgefahrene Positionen für die Elektrodenanordnung (16) bereitzustellen.

3. Elektrochirurgisches Utensil nach Anspruch 1 oder Anspruch 2, wobei die Elektrodenanordung (16) ein rückwärtiges nichtleitendes Element umfasst, das rückwärtig von dem länglichen Körper (10) vorragt und das vorwärts gedrückt werden kann, um die Elektrodenanordnung (16) in ihre ausgefahrene Position oder Positionen zu bewegen.

4. Elektrochirurgisches Utensil nach einem der vorhergehenden Ansprüche, wobei die Einrastanordnung zusammenwirkende Elemente umfasst, die mit der Elektrodenanordnung (16) bzw. dem länglichen Körper (10) assoziiert sind.

5. Elektrochirurgisches Utensil nach Anspruch 4, wobei die zusammenwirkenden Elemente auf Elementen ausgebildet sind, die an der Elektrodenanordnung (16) und dem länglichen Körper (10) angebracht sind.

6. Elektrochirurgisches Utensil nach einem der vorhergehenden Ansprüche, wobei die Vorspannungsanordnung eine Druckfeder (46) umfasst.

## Revendications

1. Instrument électrochirurgical comprenant :
un corps allongé (10) ayant une ouverture au niveau d'une extrémité avant et comprenant une partie de cylindre arrière rotative (10³) et un pressage (54) qui est fixé à la fois en rotation et longitudinalement par rapport à la partie de cylindre arrière (10³) ;
un ensemble d'électrodes (16) comprenant une électrode de traitement à travers laquelle un courant de traitement peut être passé en utilisation, l'électrode de traitement ayant une région de contact de patient au niveau d'une extrémité avant ;
un porte-électrode (18) qui porte l'ensemble d'électrodes (16) dans ledit corps (10), le porte-électrode (18) étant mobile entre une position rétractée dans laquelle la région de contact de patient de l'électrode est protégée à l'intérieur dudit corps allongé (10) et au moins une position étendue dans laquelle ladite région de contact de patient fait saillie à travers ladite ouverture ;
un agencement de sollicitation pour solliciter vers l'arrière ledit ensemble d'électrodes (16) vers sa position rétractée, et
dans lequel l'instrument électrochirurgical comprend en outre un agencement de verrouillage pour verrouiller le porte-électrode (18) comprenant une dent de verrouillage arrière (44) pouvant venir en prise avec au moins une ouverture (48, 50, 52) du pressage (54), l'agencement de verrouillage maintenant le porte-électrode (18) dans l'au moins une position étendue lorsqu'un utilisateur appuie vers l'arrière sur une partie exposée du porte-électrode (18) ; et
le cylindre arrière (10³) pouvant tourner par rapport au reste du corps allongé pour pousser la dent de verrouillage arrière (44) en alignement avec une fente de découpe longitudinale (56) dans le pressage (54), pour déverrouiller le porte-électrode (18) pour un mouvement de rétraction vers l'arrière sous l'influence de l'agencement de sollicitation.

2. Instrument électrochirurgical selon la revendication 1, l'instrument électrochirurgical étant conçu pour fournir deux positions étendues ou plus pour ledit ensemble d'électrodes (16).

3. Instrument électrochirurgical selon la revendication 1 ou la revendication 2, dans lequel ledit ensemble d'électrodes (16) comprend un élément non conducteur arrière qui fait saillie vers l'arrière à partir du corps allongé (10) et qui peut être poussé vers l'avant pour déplacer l'ensemble d'électrodes (16) vers sa position ou ses positions étendue(s).

4. Instrument électrochirurgical selon l'une quelconque des revendications précédentes, dans lequel l'agencement de verrouillage comprend des éléments coopérants associés respectivement à l'ensemble d'électrodes (16) et audit corps allongé (10).

5. Instrument électrochirurgical selon la revendication 4, dans lequel lesdits éléments coopérants sont formés sur des éléments fixés à l'ensemble d'électrodes (16) et au corps allongé (10).

6. Instrument électrochirurgical selon l'une quelconque des revendications précédentes, dans lequel l'agencement de sollicitation comprend un ressort de compression (46).
